# EUROPÄISCHE PATENTANMELDUNG

(11) **EP 1 889 596 A1**
(43) Veröffentlichungstag der Anmeldung: **20.02.2008**
(21) Anmeldenummer: 07014359.9
(22) Anmeldetag: 21.07.2007
(51) Int. Cl.: A61K 8/06, A61K 8/34, A61K 8/92, A61Q 1/02, A61Q 19/00

(54) **Kosmetische Zubereitung**

(30) Priorität: 28.07.2006 DE 102006035042
(71) Anmelder: Beiersdorf AG, 20253 Hamburg (DE)
(72) Erfinder: Kruse, Uta, 20149 Hamburg (DE); Riedel, Heidi, 22083 Hamburg (DE); Warnke, Katja Dr., 22547 Hamburg (DE); Fischer, Britta, 22880 Wedel (DE)

(57) **Zusammenfassung**

Kosmetische Zubereitung vom Typ Öl-in-Wasser, welche
d) ein oder mehrere Polyole,
e) leichte Öle mit einer Viskosität von 1 bis 15 mPa.s und einem Spreitwert von 800 bis 1200 mm²/10 min,
f) mittelschwere Öle mit einer Viskosität von 20 bis 100 mPa.s und einem Spreitwert von 300 bis 600 mm²/10 min sowie Wachse mit einem Schmelzpunkt von 25 bis 45°C

enthält, wobei die Gesamtmenge der leichten Öle kleiner ist als die Gesamtmenge an mittelschweren Ölen und Wachsen.

## Beschreibung

Die vorliegende Erfindung betrifft kosmetische Zubereitungen auf der Basis von Öl-in Wasser-Formulierungen, welche sich durch einen erhöhten Gehalt an Polyolen auszeichen. Insbesondere betrifft die vorliegende Erfindung kosmetische Zubereitungen zum Schminken der Haut.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Im Bereich der kosmetischen Mittel sind zur Färbung des Produktes oder zur Färbung des zu behandelnden "Objektes" (Haut, Haare, Lippen) sowohl lösliche Farbmittel (im Rahmen der vorliegenden Beschreibung auch als Farbstoffe bezeichnet) und unlösliche Farbmittel (im Rahmen der vorliegenden Beschreibung auch als (Farb-) Pigmente bezeichnet) zugelassen.

Alle dekorativen Körperpflegemittel enthalten einen mehr oder weniger großen Anteil an Farbstoffen, Farbpigmenten und/oder Perlglanzpigmenten, da die farbliche Veränderung der Gesichtshaut, der Augenregion, der Lippen und/oder der Nägel der Hauptzweck dieser Produkte ist. Daneben enthalten diese Produkte üblicherweise zusätzlich weitere Inhaltsstoffe mit hautpflegender oder hautschützender Wirkung. Die Anwendung kosmetischer Make-up-Zubereitungen soll im allgemeinen die Besonderheit und Individualität einer Person unterstreichen und dabei der Betonung der persönlichen Attraktivität und der Kaschierung eventuell vorkommender Makel dienen.

Natürlich ist dem Fachmann eine Vielzahl von Möglichkeiten bekannt, stabile O/W-Zubereitungen zur kosmetischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind, wobei solche Zubereitungen des Standes der Technik selbstverständlich auch Polyole enthalten können.

Ein Nachteil von polyolhaltigen O/W-Emulsionen des Standes der Technik ist aber, dass diese - sofern Polyolmengen eingesetzt werden, welche in bezug auf die Hautbefeuchtung eine gewisse Wirksamkeit zeigen - eine deutliche Klebrigkeit aufweisen und sich nur schlecht auf der Haut verteilen lassen. Um die Klebrigkeit polyolhaltiger O/W-Emulsionen zu verringern und ein leichtes Hautgefühl zu erreichen (leicht verteilbar, kaum Rückstand, schnell einziehend), wird üblicherweise mit sehr dünnflüssigen Ölen gearbeitet (d. h. mit Ölen mit einer Viskosität von 1 bis 15 mPa.s), die ferner eine sehr hohe Spreitfähigkeit (800 bis 1200 mm²/10 min) aufweisen.

Aufgabe der vorliegenden Erfindung war es daher, den Stand der Technik um hautpflegende Zubereitungen zu bereichern, welche insbesondere die Hautfeuchtigkeit verbessern und sich sehr leicht auf der Haut verteilen lassen, schnell einziehen und dabei gehaltvoll und pflegend sein sollten.

Überraschend hat sich gezeigt, dass eine
kosmetische Zubereitung vom Typ Öl-in-Wasser, welche
a) ein oder mehrere Polyole,
b) leichte Öle mit einer Viskosität von 1 bis 15 mPa.s und einem Spreitwert von 800 bis 1200 mm²/10 min,
c) mittelschwere Öle mit einer Viskosität von 20 bis 100 mPa.s und einem Spreitwert von 300 bis 600 mm²/10 min sowie Wachse mit einem Schmelzpunkt von 25 bis 45°C
   enthält, wobei die Gesamtmenge der leichten Öle kleiner ist als die Gesamtmenge an mittelschweren Ölen und Wachsen,
diese Aufgaben zu lösen vermag.

Der Spreitwert der eingesetzten Öle läßt sich ermitteln, indem 20µl des zu untersuchenden Öls in die Mitte eines Rotbandfilters punktuell aufgetragen und der Radius des gespreiteten Weges des Öls nach 10 min abgelesen werden, woraus anschließend die Fläche berechnet wird [mm²/10 min].

Die erfindungsgemäßen Zubereitungen stellen in jeglicher Hinsicht überaus befriedigende Präparate dar. Es war für den Fachmann nicht vorauszusehen gewesen, daß sich Emulsionen, welche sich durch hohe Anteile an höher viskosen, fettigen und nur wenig spreitenden Ölen und Wachsen mit einem Schmelzpunkt von 25 bis 45°C trotzdem durch ein leichtes Hautgefühl und eine deutlich verringerte Klebrigkeit auszeichnen und sich insbesondere sehr leicht auf der Haut verteilen lassen, schnell einziehen und dennoch sehr gehaltvoll und pflegend sein würden.

Die Verwendung erfindungsgemäßer Zubereitungen mit einem kosmetisch wirksamen Gehalt an einem oder mehreren Polyolen - z. B. in Form der genannten Beispiele - führt in überraschender Weise zu einer erheblichen Verbesserung des Aussehens der Haut, insbesondere werden durch Anwenden erfindungsgemäßer Zubereitungen
- ein deutlicher Schimmereffekt auf der Haut erzeugt und
- ein strahlender natürlicher Teint erreicht sowie
- die Barriereeigenschaften der Haut erhalten oder wiederhergestellt,
- besser der Hautaustrocknung entgegengewirkt und
- die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an einem oder mehreren Polyolen enthalten, zur Steigerung der Hautfeuchtigkeit bzw. zur Befeuchtung der Haut.

Die Hautfeuchtigkeit läßt sich mit Hilfe von Corneometermessungen ermitteln. Das Verfahren wird im folgenden beispielhaft stichpunktartig erläutert:
- Akklimatisierung von mindestens 30 Minuten unter Standardklimabedingungen (21,5 ± 1 °C und 45 ± 5 % relativer Luftfeuchte),
- 1. Messung vor Anwendung,
- einmalige, kontrollierte Anwendung,
- 2. Messung zwei Stunden nach der Anwendung.

Zur Anwendung werden die erfindungsgemäßen Zubereitungen in der für Kosmetika üblichen Weise auf die Haut in ausreichender Menge aufgebracht. Sie können wie üblich zusammengesetzt sein und zur Pflege der Haut und als Schminkprodukt in der dekorativen Kosmetik dienen.

Die kosmetischen Zubereitungen gemäß der Erfindung stellen bevorzugt Emulsionen dar.

Bevorzugtes Polyol im Sinne der vorliegenden Erfindung ist Glycerin, Butylen Glykol, Propylen Glykol.

Erfindungsgemäß bevorzugte leichte Öle sind solche mit den INCI-Bezeichnungen Isodecyl Neopentanoate, Dicaprylyl Ether, Isoeikosan, Propylene Glycol Dicaprylate/Dicaprate, Coco Dicaprylate/Dicaprate, Butylene Glycol Dicaprylate/Dicaprate, Cyclomethicone, C13-C16 Isoparaffin, Isohexadecane, Octyl Palmitate, Dicaprylyl Carbonate. Besonders bevorzugt sind Cyclomethicone, Isohexadecane, Dicaprylyl Carbonate, Dicapyrlyl Ether.

Es ist vorteilhaft im Sinne der vorliegenden Erfindung die Konzentration der leichten Öle - bezogen auf das Gesamtgewicht der Zubereitung - aus dem Bereich von 5 bis 15 Gew.-%, bevorzugt von 10 bis 12 Gew.-% zu wählen.

Erfindungsgemäß bevorzugte mittelschwere Öle sind Mineralöle und/oder solche mit den INCI-Bezeichnungen Cocoglyceride, Caprylic /Capric Triglyceride, Polydecene, Dipentaerythrityl Hexacaprylate/Hexacaprate, Dimethicone, Squalane. Besonders bevorzugt sind Caprylic /Capric Triglyceride, Dimethicone, Squalane.

Es ist vorteilhaft im Sinne der vorliegenden Erfindung die Konzentration der mittelschweren Öle - bezogen auf das Gesamtgewicht der Zubereitung - aus dem Bereich von 2,5 bis 20 Gew.-%, bevorzugt von 7,5 bis 15 Gew.-%, besonders bevorzugt von 12,5 bis 15 Gew.-% zu wählen.

Erfindungsgemäß bevorzugte Wachse mit Schmelzpunkten von 25 bis 45°C sind solche mit den INCI-Bezeichnungen Hydrogenated Cocoglycerides, Mristyl Myristate, Myristyl Lactate, Caprylic/Capric/Isostearic/Adipic Triglyceride, Shea Butter, Stearyl Dimethicone, Hydrogenated Vegetable Oil. Besonders bevorzugt sind Shea Butter, Myristyl Lactate.

Es ist vorteilhaft im Sinne der vorliegenden Erfindung die Konzentration dieser Wachse - bezogen auf das Gesamtgewicht der Zubereitung - aus dem Bereich von 1 bis10 Gew.-%, bevorzugt von 2,5 bis 7,5 Gew.-%, besonders bevorzugt von 2,5 bis 5 Gew.-% zu wählen.

Es ist vorteilhaft im Sinne der vorliegenden Erfindung, wenn das Verhältnis der Gesamtmengen von leichten Ölen einerseits zu mittelschweren Ölen und Wachsen andererseits von 1 : 1 bis 1 : 2 gewählt wird.

Die erfindungsgemäßen Zubereitungen enthalten vorteilhaft einen oder mehrere O/W-Emulgatoren, vorzugsweise aus der folgenden Gruppe:

Sucroseester - wie insbesondere Sucrosepalmitat - in Kombination mit Glycerylstearat und Glycerylstearatcitrat (beispielsweise erhältlich unter dem Handelsnamen Arlatone V-175 von der Fa. Uniqema), Glycerylstearat in Kombination mit Ceteareth-20 und/oder Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Isoceteth-20, Glycerylstearat in Kombination mit Ceteareth-20, Ceteareth-12, Cetylstearylalkohol und/oder Cetylpalmitat, Cetylstearylalkohol in Kombination mit PEG-20 Stearat, PEG-30-Stearat, PEG-40-Stearat und/oder PEG-100-Stearat sowie Hydrogenated Lecithin.

Erfindungsgemäß besonders vorteilhafte O/W-Emulgatoren sind Sucroseester - wie insbesondere Sucrosepalmitat - in Kombination mit Glycerylstearat und Glycerylstearatcitrat (beispielsweise erhältlich unter dem Handelsnamen Arlatone V-175 von der Fa. Uniqema) Glycerylstearat, PEG-40 Stearat, Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol und/oder Sorbitanstearat sowie Hydrogenated Lecithin.
Ganz besonders bevorzugter Emulgator im Sinne der vorliegenden Erfindung ist der mit der INCI-Bezeichnung Hydrogenated Lecithin, welcher beispielsweise unter der Handelsbezeichnung Biophilic H von der Fa. Lucas Meyer erhältlich ist und eine Abmischung aus C12-C16 Alcohol, Hydriertem Lecithin und Palmitinsäure darstellt.

Die erfindungsgemäßen Zubereitungen können vorteilhaft auch anorganische oder organische Pigmente enthalten.

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. EisenoxidPigmente, Chromoxide), wobei die Pigmente beschichtet ("gecoatet") oder unbeschichtet vorliegen können. Unter den Farbpigmenten sind Eisenoxide besonders bevorzugt.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im Allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß-und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelübliche Effektpigmente sind: Timiron® von Merck , Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyl Lysine, Polymethylsilesquioxane, Polymethylmethacrylate, Polymethylmethacrylate Crosspolymer, Nylon, Talkum, beschichtetes Talkum, z.B. mit Dimethicone und Trimethylsiloxysilicate, Mica, Silica

Feine Fältchen sieht man, weil sich die Lichtreflexion auf der Hautoberfläche von der in den Faltensenken unterscheidet. Es ist daher vorteilhaft im Sinne der vorliegenden Erfindung, lichtstreuende Pigmente einzusetzen, die die Faltentiefe optisch reduzieren, indem sie den Anteil an diffus reflektiertem Licht in der Falte reduzieren, wodurch die Haut glatter und natürlicher aussieht. Auf diese Weise lässt sich auch übermäßiger Glanz der Haut optisch reduzieren. Vorteilhaft zu diesem Zweck zu verwenden ist speziell beschichtetes Silica - z. B. ganz besonders vorteilhaft Silica LDP (Silica + Titanium Dioxide + Iron Oxides), welches unter dem Handelsnamen Ronasphere LDP von Merck erhältlich ist.

Die kosmetischen Zubereitungen gemäß der Erfindung können ferner vorteilhaft einen oder mehrere grenzflächenaktive Polyether enthalten:

Vorteilhaft im Sinne der vorliegenden Erfindung sind die in den Chemical Abstracts mit der Registraturnummer 78336-31-9 bezeichneten Polyether, welche die chemische Bezeichnung Polyethylenglycoldi(polydodecylenglycol)ether tragen und beispielsweise als PEG-45 (dodecyl glycol) copolymer unter der Marke Elfacos® ST 9 (mittleres Molekulargewicht ca. 4 000 g/mol) bzw. als PEG-22 (dodecyl glycol) copolymer unter der Marke Elfacos® ST 37 (mittleres Molekulargewicht ca. 2 300 g/mol) von der Gesellschaft Akzo Nobel Chemicals GmbH verkauft werden.

Die Gesamtmenge an den grenzflächenaktiven Polyethern in den fertigen kosmetischen Zubereitungen wird vorteilhaft aus dem Bereich von 0,1 bis 30 Gew.-%, bevorzugt 0,25 bis 5,0 Gew.-% insbesondere 0,75 bis 3,5 Gew.-% gewählt, jeweils bezogen auf das Gesamtgewicht der Zubereitungen.

Es ist vorteilhaft im Sinne der vorliegenden Erfindung, wenn die Zubereitungen einen oder mehrere Glycerinester, insbesondere Glycerinester von α-Hydroxycarbonsäuren und gesättigten Fettsäuren enthalten, wobei die Gesamtmenge der Glycerinester in den fertigen kosmetischen Zubereitungen vorteilhaft aus dem Bereich von 0,1 bis 10,0 Gew.-%, bevorzugt 0,5 bis 6,0 Gew.-% gewählt wird, jeweils bezogen auf das Gesamtgewicht der Zubereitungen.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Siliconderivate.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure (Vitamin C) und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E oder Vitamin C und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. Ginkgo Biloba bzw. Ginkgo-Extrakte, Hyaluronsäure, Collagen, alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCl-Bezeichnung Octadecendioic acid).

Erfindungsgemäße Zubereitungen, welche z. B. bekannte Faltenfüllwirkstoffe wie Hyaluronsäure und/oder Derivate und dergleichen enthalten, eignen sich insbesondere vorteilhaft zur kosmetischen Behandlung von Hautveränderungen, wie sie z. B. bei der Hautalterung auftreten (wie Ausbildung von Trockenheitsfältchen, Schlaffheit und Ausbildung von Falten und Fältchen). Weiterhin vorteilhaft eignen sich Collagen bzw. Wirkstoffe, die die Collagensynthese steigern, wie Ginkgo Biloba bzw. Ginkgo-Extrakte, Vitamin C, Creatin, um die Haut zu festigen und zu straffen.

Vorteilhaft ist ferner die Verwendung von erfindungsgemäßen Zubereitungen, welche Ascorbinsäure und/oder Derivate und/oder UV-Filtersubstanzen enthalten, zur kosmetischen Behandlung von Hautalterungserscheinungen (Ausbildung von Trockenheitsfältchen, Schlaffheit und Ausbildung von Falten und Fältchen).

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Magnesium Aluminium Silikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Pemulen TR-1 oder -2, Ultrez 10, jeweils einzeln oder in Kombination.

Auch sog. Moisturizer sind vorteilhaft zu verwenden. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Es ist erfindungsgemäß bevorzugt, den Gehalt an UV-Filtersubstanzen (eine oder mehrere Verbindungen) kleiner als 5 Gew.-%, insbesondere kleiner als 2 Gew.-% zu wählen, jeweils bezogen auf das Gesamtgewicht der Zubereitung.

Für den Fall, dass ein Gehalt an UV-Filtersubstanzen gewünscht ist, werden diese vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen. Anorganische Pigmente wie Titandioxid besonders bevorzugt Titandioxid AQ-IP der Fa. Unichema.

Besonders vorteilhafte UV-Filtersubstanzen im Sinne der vorliegenden Erfindung sind:
- Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird;
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird;
- Benzol-1,4-di(2-oxo-3-bomylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.
- Benzoxazol-Derivate, wie z. B. das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist;
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist;
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCl: Methylene Bis-Benztriazolyl Tetramethylbutylphenol), welches z. B. unter der Handelsbezeichnung Tinosorb® M bei der CIBA-Chemikalien GmbH erhältlich ist;
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester (Octylmethoxycinnamate), 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- an Polymere gebundene UV-Filter sowie
- Ethylhexyl-2-cyano-3,3-diphenylacrylat (Octocrylen), welches von BASF unter der Bezeichnung Uvinul^{®} N 539 T erhältlich ist.

Es ist ferner vorteilhaft im Sinne der vorliegenden Erfindung, wenn die Zubereitung einen oder mehrere Filmbildner bzw. Polymere enthält, insbesondere um einen nachvollziehbaren Straffungseffekt auf der Haut zu erzielen, der durch deren Filmbildungseigenschaften hervorgerufen werden kann. Gleichzeitig dienen die Filmbildner zur Fixierung der Pigmente auf der Haut, insbesondere um einen langanhaltenden Effekt und eine Transfer-Resistenz zu erzielen.

Vorteilhafte Filmbildner im Sinne der vorliegenden Erfindung sind z. B. Trimethylsiloxysiliacte (beispielsweise auch in Abmischung mit Dimethicone), Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), C₂₀₋₄₀ Carbonsäure mit Polyethylen (Performacid 350 von der Fa. New Phase Technologies) sowie Filmbildner aus der Gruppe der Polymere auf Basis von Polyvinylpyrrolidon (PVP):

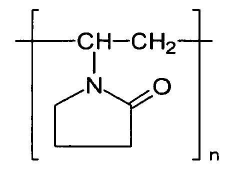

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Insbesondere bevorzugt sind wasserlösliche Polymere wie VinylpyrrolidonNinylacetat- (VP/VA-) Copolymere und Natriumpolystyrensulfonat.

Es ist vorteilhaft im Sinne der vorliegenden Erfindung die Konzentrationen an Filmbildnern (eine oder mehrere Verbindungen) aus dem Bereich von 0,1 bis 5 Gew.-%, besonders bevorzugt wie 0,1 bis 3 Gew.-% - jeweils bezogen auf das Gesamtgewicht der Zubereitungen - zu wählen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| **O/W Foundation** | | | | | | | |
|---|---|---|---|---|---|---|---|
| | **1** | **2** | **3** | **4** | **5** | **6** | **7** |
| Hydriertes Lecithin, C12-C16 Fettalkohol, Palmitinsäure⁴ | | | | 3,00 | 2,00 | | 4,00 |
| Glycerinmonostearat SE | | | 3,00 | | | 1,50 | |
| Glyceryl Stearat Citrat | 2,00 | | | | | | |
| Stearinsäure | | | 0,75 | | | | |
| PEG-40 Stearat | | | | | | 2,00 | |
| PEG-100 Stearat | | | 1,50 | | | | |
| Cetyl Phosphat | | | 0,75 | | | | |
| Stearyl Alkohol | | | 3,00 | | | 2,00 | |
| Cetyl Alkohol | 2,50 | | | | | | |
| Sucrose Palmitate ¹ | | 2,00 | | | | | |
| Bis-Ethylhexyloxyphenol Methoxyphenyl Triazin | | 1,00 | | | | | |
| Disodium Phenyl Dibenzimidazol Tetrasulfonat | | | | 1,00 | | | |
| Ethylhexyl Triazon | 2,00 | | | | | | |
| Ethylhexyl Methoxycinnamat | | 3,50 | | 5,00 | | | |
| Octocrylen | | | | | | | 2,50 |
| Titandioxid T 805 | | 1,50 | | | 1,00 | | |
| Titandioxid MT-100Z | 1,00 | | | | 1,00 | | |
| C₁₂₋₁₅ Alkyl Benzoat | | | | | | 7,00 | |
| Dicaprylylether | | | 3,50 | | 1,00 | 4,00 | |
| Butylenglycol Dicaprylat/ Dicaprat | 2,00 | | 1,50 | 2,00 | 1,00 | | 2,50 |
| Caprylic/Capric Triglycerid | | 3,50 | 2,00 | 4,00 | 2,00 | 4,00 | 2,00 |
| Cetearyl Isononanoate | | 2,00 | | | | 2,00 | 2,00 |
| Dimethicon | 5,00 | 4,50 | 4,00 | 5,00 | 8,00 | 5,00 | 4,00 |
| Cyclomethicon | 5,00 | 7,00 | | 6,00 | 8,00 | 7,5 | 2,50 |
| Dimethicon / Vinyl Dimethicon Crosspolymer | 4,00 | | | | | | 0,50 |
| Squalane | | 2,00 | 1,50 | | 1,50 | 4,00 | 2,00 |
| Myristyl Lactate | 1,50 | 2,50 | 1,00 | 2,00 | | 3,50 | 2,00 |
| Shea Butter | 1,50 | 0,50 | 1,00 | 1,00 | 4,00 | | 2,00 |
| Methyl Methacrylate Crosspolymer | 1,00 | | | | | | 1,00 |
| Nylon -12 | 2,00 | | | | | 2,00 | |
| Polymethylsilesquioxane | | 2,00 | | 2,00 | 1,00 | 1,00 | |
| Lauroyl Lysine | | 3,00 | | 2,00 | 2,00 | | 1,00 |
| Titandioxid (Cl 77891) | 9,00 | 6,00 | 7,00 | 6,00 | 4,50 | 3,00 | 10,00 |
| Pigmente (Cl77492, 77491,77499,77007) | 2,0 | 3,0 | 2,6 | 4,0 | 1,5 | 2,5 | 3,0 |
| Beschichtetes Silica ² | | 0,50 | | 1,00 | | | |
| Effektpigmente (z.B. beschichtetes Mica) ³ | | 1,00 | | | | | 0,27 |
| Glycerin | 3,00 | 7,50 | | 7,50 | 5,00 | | 10,00 |
| Butylenglycol | 7,00 | 5,00 | | | | 7,00 | |
| Vitamin E Acetate | | 0,5 | 1,00 | | 0,25 | 0,4 | 0,5 |
| Vitamin A Palmitat | 0,50 | | 0,25 | 0,20 | | 0,1 | 1,00 |
| Natrium Polystyrene Sulfonate | 0,2 | | 0,25 | 0,10 | | | |
| VP/VA Copolymer | | 0,5 | | | | | 2,0 |
| PVP Hexadecen Copolymer | 0,5 | | | | 0,10 | | |
| Xanthan Gummi | 0,5 | 0,30 | 0,3 | | | 0,5 | |
| Magnesium Aluminium Silikate | | 3,00 | | | 1,00 | | 0,50 |
| DMDM Hydantoin | | 0,60 | 0,40 | 0,20 | | | |
| Parabene | 0,5 | | 0,25 | | 0,70 | | 0,70 |
| Phenoxyethanol | 1,00 | 0,40 | | 0,40 | 0,90 | | 0,60 |
| EDTA oder Tetra Sodium Iminodisuccinate | | 0,20 | 0,35 | 0,50 | 0,02 | | 0,03 |
| Ethanol | 5,00 | | 10,00 | | 3,00 | | |
| Parfüm | 0,20 | 0,20 | | | | 0,30 | 0,40 |
| Wasser | ad 100 | ad 100 | ad 100 | ad 100 | ad 100 | ad 100 | ad 100 |

| | | | | | | | |
|---|---|---|---|---|---|---|---|
| ¹ Arlatone V-175 von Uniqema (Phenoxyethanol + Methylparaben+ Ethylparaben + Butylparaben + Isobutylparaben+ Propylparaben + Sucrose Palmitate + Glyceryl Stearate + Sucrose + Xanthan Gum + Mannan + Glyceryl Stearate Citrate) ^{2 z.B.} Ronasphere LDP von Merck (Silica + Titanium Dioxide + Iron Oxides) ^{3 z.B..} Timiron von Merck (Mica & Cl 77891) ⁴ Biophilic H von der Fa. Lucas Meyer | | | | | | | |

## Patentansprüche

1. Kosmetische Zubereitung vom Typ Öl-in-Wasser, welche
a) ein oder mehrere Polyole,
b) leichte Öle mit einer Viskosität von 1 bis 15 mPa.s und einem Spreitwert von 800 bis 1200 mm²/10 min,
c) mittelschwere Öle mit einer Viskosität von 20 bis 100 mPa.s und einem Spreitwert von 300 bis 600 mm²/10 min sowie Wachse mit einem Schmelzpunkt von 25 bis 45°C
enthält, wobei die Gesamtmenge der leichten Öle kleiner ist als die Gesamtmenge an mittelschweren Ölen und Wachsen.

2. Zubereitung nach Anspruch 1, **dadurch gekennzeichnet, dass** als Polyol Glycerin gewählt wird.

3. Zubereitung nach einem der vorhergehenden Ansprüche, **dadurch gekennzeichnet, dass** das oder die leichten Öle aus der Gruppe Isodecyl Neopentanoate, Dicaprylyl Ether, Isoeikosan, Propylene Glycol Dicaprylate/Dicaprate, Coco Dicaprylate/Dicaprate, Butylene Glycol Dicaprylate/Dicaprate, Cyclomethicone, C13-C16 Isoparaffin, Isohexadecane, Octyl Palmitate, Dicaprylyl Carbonate gewählt werden.

4. Zubereitung nach einem der vorhergehenden Ansprüche, **dadurch gekennzeichnet, dass** die Konzentration der leichten Öle - bezogen auf das Gesamtgewicht der Zubereitung - aus dem Bereich von 5 bis 15 Gew.-% gewählt wird.

5. Zubereitung nach einem der vorhergehenden Ansprüche, **dadurch gekennzeichnet, dass** das oder die mittelschweren Öle aus der Gruppe der Mineralöle und/oder Cocoglyceride, Caprylic/Capric Triglyceride, Polydecene, Dipentaerythrityl Hexacaprylate/Hexacaprate, Dimethicone, Squalane gewählt werden.

6. Zubereitung nach einem der vorhergehenden Ansprüche, **dadurch gekennzeichnet, dass** die Konzentration der mittelschweren Öle - bezogen auf das Gesamtgewicht der Zubereitung - aus dem Bereich von 7,5 bis 15 Gew.-% gewählt wird.

7. Zubereitung nach einem der vorhergehenden Ansprüche, **dadurch gekennzeichnet, dass** das oder die Wachse mit einem Schmelzpunkt von 25 bis 45°C aus der Gruppe Hydrogenated Cocoglycerides, Mristyl Myristate, Myristyl Lactate, Caprylic/Capric/Isostearic/Adipic Triglyceride, Shea Butter, Stearyl Dimethicone, Hydrogenated Vegetable Oil gewählt werden.

8. Zubereitung nach einem der vorhergehenden Ansprüche, **dadurch gekennzeichnet, dass** die Konzentration der Wachse - bezogen auf das Gesamtgewicht der Zubereitung - aus dem Bereich von 1 bis10 Gew.-% gewählt wird.

9. Zubereitung nach einem der vorhergehenden Ansprüche, **dadurch gekennzeichnet, dass** sie zusätzlich Pigmente enthält.

10. Zubereitung nach Anspruch 9, **dadurch gekennzeichnet, dass** Pigmente Effektpigmente verwendet werden.

11. Zubereitung nach einem der vorhergehenden Ansprüche, **dadurch gekennzeichnet, dass** sie einen oder mehrere Füllstoffe enthält.
